# EUROPEAN PATENT SPECIFICATION

(11) **EP 0 934 431 B1**
(45) Date of publication and mention of the grant of the patent: **18.12.2002**
(21) Application number: 97909100.6
(22) Date of filing: 17.10.1997
(51) Int. Cl.: C12Q 1/48, C12N 1/20

(54) **METHOD AND CELLCULTURE FOR IDENTIFYING INHIBITORS OF EUKARYOTIC CELL PROCESSES**
VERFAHREN UND ZELLKULTUR ZUR IDENTIFIZIERUNG VON INHIBITOREN VON EUKARIOTISCHEN ZELLAKTIVITÄTEN
PROCEDE ET CULTURE CELLULAIRE PERMETTANT D'IDENTIFIER DES INHIBITEURS DE L'ACTIVITE DES CELLULES EUCARYOTES

(30) Priority: 17.10.1996 US 733686
(43) Date of publication of application: 11.08.1999
(73) Proprietor: Terragen Diversity Inc., Vancouver, British Columbia V6T 1Z3 (CA)
(72) Inventor: DAVIES, Julian, E., Vancouver, British Columbia V6R 1V3 (CA); WATERS, Barbara, Delta, British Columbia V4L 2E6 (CA); SAXENA, Geeta, Vancouver, British Columbia V6T 2G3 (CA)
(74) Representative: Hammond, Andrew
(86) International application number: PCT/CA97/00781
(87) International publication number: WO 98/017822

(56) References cited:
- US-A- 5 143 839
- US-A- 5 451 518
- WATERS B ET AL: "Protein tyrosine phosphorylation in streptomycetes." FEMS MICROBIOLOGY LETTERS 120 (1-2). 1994. 187-190. ISSN: 0378-1097, XP002053919
- HONG S-K ET AL: "EFFECTS OF PROTEIN KINASE INHIBITORS ON IN-VITRO PROTEIN PHOSPHORYLATION AND CELLULAR DIFFERENTIATION OF STREPTOMYCES - GRISEUS." MOL GEN GENET 238 (2-3). 1993. 347-354. CODEN: MGGEAE ISSN: 0026-8925, XP002053920

## Description

### BACKGROUND OF THE INVENTION

This application relates to a method and microbial composition for assaying materials for activity as inhibitors of kinase and phosphatase enzymes.

Kinase and phosphatase enzymes play important roles in the regulation of both eukaryotic and prokaryotic cells. For example, in eukaryotic cells, the control of proliferation and differentiation is achieved by multiple signal transduction pathways that are regulated by the coordinated action of protein kinases and phosphatases. Prokaryotic cells also rely on protein phosphorylation cascades for regulation of cellular activities. These kinases, and their associated response regulators are involved in adaptive responses such as nitrogen fixation, chemotaxis in enteric bacteria and regulation of sporulation in *Bacillus* species.

Kinase activity in eukaryotes can be classified as one of three types: those enzymes which phosphorylate tyrosine residues; those which are specific for serine or threonine residues; and those which have dual specificity for both tyrosine and serine/threonine residues. Because of the importance of these enzymes in eukaryotic regulatory processes, it would be highly desirable to be able to inhibit kinases of the various classes selectively to assist in the elucidation of kinase and phosphatase mediated pathways, particularly those that may be of medical significance. In addition, selective kinase or phosphatase inhibitors have potential uses as therapeutics. For example, it has been reported that a tyrosine kinase blocker designated AG-940 specifically inhibits the Jak-2 protein tyrosine kinase which is deregulated and constitutively activated in the leukemic cells of acute lymphoblastic leukemia (ALL) patients. Meydan, et al. "Inhibition of acute lymphoblastic leukaemia by a Jak-2 inhibitor", *Nature* 379: 645-648 (1996). This inhibition induced changes in cells consistent with entry into apoptosis when tested *in vitro.* Further, the intravenous administration of the inhibitor into mice previously injected with ALL cells has been shown to be effective to eradicate the ALL cells from the marrow.

Notwithstanding the potential uses of kinase and phosphatase inhibitors, the number of known and characterized inhibitors is quite small. Staurosponne and K-252a are known to act as generalized kinase inhibitors, while herbimycin and radicol specifically inhibit tyrosine kinases, albeit with fairly low effectiveness. There are no known specific inhibitors for the MAP kinase family, an important group of enzymes thought to be central in the transmission of a wide variety of signals received at the cellular membrane to the transcriptional and replication machinery of the nucleus.

Hong et al., *Molecular Gen. Genetics* 236: 347-354 (1993) describe an *in vivo* experiment in which known eukaryotic kinase inhibitors are contacted with cultures of *Streptomyces griseus* IFO 13350 and observing the subsequent morphological changes in the cultures. This document does not contain any indication as to the development of an assay method for testing unknown substances for their potential to inhibit eukarytoic phosphorylation reactions.

To facilitate the identification of new kinase and phosphatase inhibitors, it would be very advantageous to have a simple, visually-scorable assay methodology for inhibition activity. It is an object of the present invention to provide such a method and a kit useful in practicing the method.

It is a further object of the invention to provide a microbial strain which is particularly suited for use in the present invention.

### SUMMARY OF THE INVENTION

In accordance with the present invention, a material can be assayed for activity as an inhibitor of post-translational protein phosphorylation by
adding the material to a growing culture of a prokaryotic organism such as a streptomycete;
allowing the culture to grow for a period of time in the presence of the material; and
observing the culture for altered development relative to development of the prokaryotic organism grown in the absence of the material. Observation of altered development is indicative that the material has activity as an inhibitor of post-translational protein phosphorylation. In particular, the material to be tested can be added to a growing culture of the prokaryotic organism by placing a carrier disk bearing the material on a freshly seeded plate. Inhibition of the development of aerial mycelia and spore formation is an indicator that the material has activity as an inhibitor of post-translational protein phosphorylation. This method has been used to identify four compounds isolated from microbial supernatants and lichen extracts, with the result that four known compounds have been found to have previously unrecognized effectiveness as kinase inhibitors.

### BRIEF DESCRIPTION OF THE FIGURES

Figs. 1A and 1B show the structures of viscosin and surfactin;
Fig. 2 shows the structure of vulpinic acid; and
Fig. 3 shows the structure of usnic acid.

### DETAILED DESCRIPTION OF THE INVENTION

Although eukaryotic and prokaryotic protein kinases generally have different substrate speciflcities, it has been observed that in some prokaryotic organisms eukaryote-like kinase and phosphatase activities may complement the two component systems typical of bacteria. In particular, streptomycetes, (Waters et al., "Protein tyrosine phosphorylation in streptomycetes", *FEMS Microbiology Letter* 120: 187-190 (1994); Li et al, "Cloning purification and properties of a phosphotyrosine protein phosphatase from *Streptomyces coelicolor* A3(2)", *J. Bact.* 178: 136-142 (1996)); *Myxococcus xanthus* (Zhang et al., "Identification of a putative eukaryotic-like protein kinase family in the developmental bacterium *Myxococcus xanthus", J. Bact.* 174: 5450-5453 9192); and cyanobacterial species. (Zhang, C-C, "Bacterial signaling involving eukaryotic-type protein kinases", *Molec. Microbial*. 20: 9-15 (1996)). The present invention takes advantage of this property of certain microbes to provide an assay for screening materials for activity as inhibitors of eukaryotic post-translational protein phosphorylation.

The present invention is applicable to the evaluation of any type of material for activity as an inhibitors of eukaryotic post-translational protein phosphorylation. Thus, the method of the invention can be employed to screen purified compounds. A preferred use of the invention, however, is for screening cell-free preparations derived from cultures of uncharacterized or poorly characterized microorganisms to identify those which produce natural product inhibitors of protein phosphorylation. If desired, the active inhibitor within the cell free preparation may then be isolated and purified for further characterization, testing and use.

In accordance with the present invention, the first step in assay method is adding the material to be evaluated to a growing culture of a prokaryotic test organism which possesses enzyme activity effective to phosphorylate tyrosine, serine or threonine residues within a protein. Preferably, the organism will possess both tyrosine and serine/threonine specific enzymes.

Suitable prokaryotic test organisms for use in the assay of the invention are streptomycetes, particularly strains of *Streptomyces griseus*, and a number of wild stains (e.g., strains designated as WEC93-17A, WEC188-31C, WEC362-68A and WEC403-73F) demonstrated to be distinct by sequencing of the 16S rDNA. A particularly preferred prokaryotic organism is a wild strain of *Streptomyces* isolated from soil and designated *Streptomyces* WEC478-85E (hereinafter strain 85E). This strain has been deposited with the American Type Culture Collection in accordance with the provisions of the Budapest treaty and has been assigned Accession Number ATCC 55824.

The material to be tested can be applied to a filter paper disk and then placed on a plate which has been freshly seeded with the prokaryotic test organism. The prokaryotic test organism is then allowed to grow in the presence of the filter paper disk for a period of 24 to 36 hours, after which the organism is evaluated for altered development in the zone around the disk. The effects observed may include overall growth inhibition, but at least in the case of streptomycetes, an observation of an inhibition of the formation of aerial mycelia and spores, without inhibition of the growth of vegetative mycelia is particularly indicative of the presence of an inhibitor of post-translational phosphorylation.

Depending on the specific test organism used, the growth medium employed may need to be a minimal media or a rich medium. This is the case because it appears that the metabolic pathways facilitating growth on minimal media are different from those operational during growth on rich media, and it may be the case that different kinases and phosphatases may be regulating development and metabolism under different growth conditions. It may also be advantageous to test materials using both a rich and a minimal medium.

In the case of *Streptomyces* 85E, the method of the invention is preferably performed using a minimal medium such as ISP4, an inorganic salts/starch agar available from Difco, because this strain sporulates readily when grown on this medium. This facile sporulation makes it easy to make a visual evaluation for differences in spore formation, leading to an easily scored assay. Strains (such as 17A and 31C) which sporulate on rich medium, for example tryptic soy medium, can also be used in the assay of the invention.

Materials which are identified as being or containing potential inhibitors of eukaryotic post-translational phosphorylation based upon their ability to effect the growth of the prokaryotic test organism may be further tested using one or more class specific assays to confirm and characterize the type of enzymes inhibited. For example, the materials may be tested in individual assays for inhibition of MAP kinase activity, tyrosine kinase activity, e.g. src activity, or serine/threonine kinase activity, e.g., cdc2 activity. Using the method of the invention in combination with such assays, we have identified several kinase-specific inhibitors including a MAP kinase inhibitor, src kinase and cdc2 kinase.

Although the main application for the present invention is in screening for inhibitors of post-translational phosphorylation, the bacterial assay system of the invention is in reality effective as a screening tool for inhibition of signaling pathways in eukaryotes generally. Thus, in addition to kinase and phosphatase inhibitors, the assay of the invention is useful for screening for inhibitors of cell-cycle development, inhibitors of apoptosis, inhibitors of signal mediation via calcium, and inhibitors of other eukaryotic processes that involve post-translational modification.

The assay offers a simple and effective pre-screening tool which is easily scored, and which lends itself to automated, high-throughput screening. Further, materials selected for further evaluation as a result of the assay are already known to effective in the cell, unlike activities based on *in vitro* assay systems. Most importantly, the assay system is not affected by compounds in the materials being tested that are cytotoxic to mammalian cells, thus interfering with assays using mammalian cells, and avoids problems with protease contaminants that do not interfere with microbial morphological assays but are a serious problem with animal cell and isolated receptor assays.

Once microbial cultures are identified which produce inhibitors of the type detected using the assay of the invention, the specific inhibitor that is produced by the culture may be isolated and characterized. For example, two bacterial cultures, WEC64-11C and WEC297-60A, which have been identified as strains of *Pseudomonas* and *Bacillus,* respectively, produce cell free supernatants which inhibit sporulation and formulation of aerial mycelia in tester strain 85E and other strains of *Streptomyces.* For each culture, an ethyl acetate extract was prepared by separately extracting the supernatant and the cells and combining the resulting products and the extract was then fractionated on a silica gel column. Activity in the fractions recovered from the column was assayed to permit identification of an active inhibitory fraction. This fraction was further purified by chromatography, and the inhibitory compound was analyzed by mass spectroscopy.

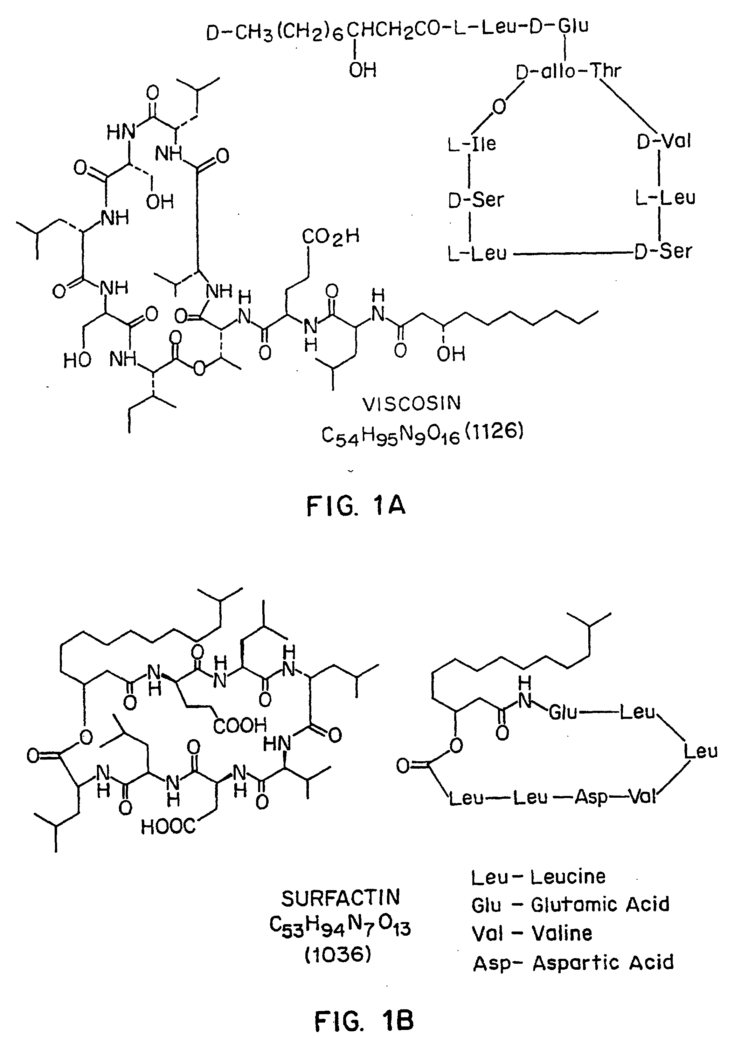

In the case of the *Pseudomonas* 11C, the product was determined to be viscosin. In the case of *Bacillus* 60A, the product was determined to be surfactin. The structures of viscosin and surfactin are shown in Figs. 1A and 1B. Both are lipopeptide biosurfactants which are synthesized non-ribosomally by multi-functional peptide synthetases. (Stachelhaus et al., *Biochemical Pharmacology* 52: 177-186 (1996). Tests using pure viscosin and surfactin obtained from third-party sources confirmed the ability of these compounds to act as inhibitors of human c-Src kinase, a tyrosine kinase and to a lesser extent as inhibitors of the phosholipid dependent serine-threonine kinase, Protein Kinase C (PKC).

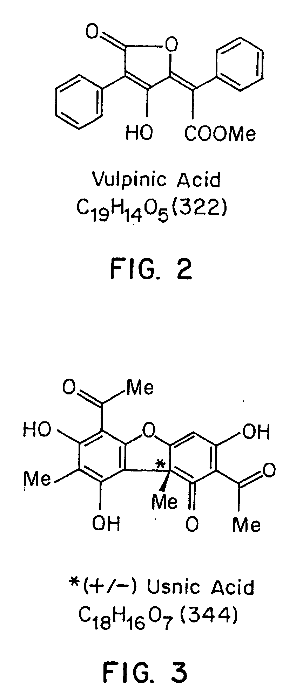

The method of the invention has also been used to screen extracts from 62 species of lichens, of which 52 extracts were found to inhibit sporulation of tester strain *Streptomyces* 85E. Because of the breadth of inhibitory results found, two widely distributed lichen compounds, vulpinic acid (Fig. 2) and usnic acid (Fig. 3) were obtained in purified form and tested for activity as inhibitors of sporulation in the first instance and then as kinase inhibitors.

Usnic acid is a widely distributed lichen compound. Huneck and Yoshimura, *Identification of Lichen Substances,* Springer-Verlag, Berlin (1996) list 16 lichen genera from which this compound has been isolated. Vulpinic acid is usually isolated from *Letharia vulpina*, but is also found in other lichen species. Both of these compounds have been reported to have antibacterial activities (Lauterwein *et al., Antimicrobial Agents and Chemotherapy* 39: 2541-2543 (1995)) but neither has previously been identified as a kinase inhibitor. Both of the purified compounds were found to inhibit sporulation in *Streptomyces* 85E. Both compounds were also found to inhibit human c-Src kinase, although to a lesser extent than surfactin or viscosin. Minimal inhibition of Protein Kinase C was also observed for both compounds. Although the kinase inhibitory activity was not particularly potent with respect to the two enzymes tested, the sporulation inhibition activity is very marked and it is possible that other eukaryotic kinases may be more strongly affected by these compounds.

These results demonstrate the ability of the method of the present invention to identify compounds with therapeutic potential as inhibitors of cell processes. Using the methods of the invention, four compounds were identified which had been previously unrecognized as having inhibitory properties. Given the potential importance of such inhibitors as therapeutics, and the small numbers of inhibitors now known, these results confirm the importance of the present invention for providing rapid screening of new therapeutic candidates from among compounds with previously unrecognized inhibitory properties.

The invention will now be further described and illustrated by way of the following, non-limiting examples.

### EXAMPLE 1

A screening assay was run on a total of 1000 soil sample isolates using *Streptomyces* 85E as the prokaryotic test organism. Each isolate was grown in tryptic soy broth (Difco) for a period of 2-3 days. A cell-free culture supernatant was then collected by centrifugation at 17,000 X g for 10 minutes. 30 µl of this supernatant was dispensed onto a 12 mm diameter filter paper disk. The disks were then placed on plates prepared from ISP4 minimal medium and freshly seeded with *Streptomyces* 85E. The plates were incubated for 24 to 36 hours at 30C in a standard incubator cabinet. During this time, the cultures were observed for differences in growth and/or development in the areas surrounding the disk.

Fifty-two of the 1000 isolates, including species of *Streptomyces, Bacillus* and *Pseudomonas,* were found to produce a supernatant which affects the sporulation of the indicator strain *Streptomyces* 85E.

In a follow-up experiment, this test was repeated using 10-fold and 100-fold dilutions of the original supernatant for three of the stronger inhibitors. Sporulation of the indicator strain *Streptomyces* 85E was seen to be reduced with the 10-fold dilution but could not be detected with the 100-fold dilution.

### EXAMPLE 2

Thirty seven of the supernatants found to inhibit sporulation of *Streptomyces* 85E were tested for their ability to inhibit sporulation in *Streptomyces griseus* ATCC No. 23345 using the same procedure described in Example 1. Sixteen of the supernatants were found to inhibit sporulation of this strain. Thus, *Streptomyces griseus* ATCC No. 23345 can be used in the assay of the invention, although it is less sensitive than *Streptomyces* 85E.

### EXAMPLE 3

The fifty two supernatants found to inhibit sporulation of *Streptomyces* 85E were tested for their ability to inhibit growth of a gram positive bacterium (*S. aureus* RN450), a gram negative bacterium (*E. coli* strain DB10), and a yeast species (*S. cerevisiae* strain RC1-707). Most did not affect growth of the bacterial species. A few had anti-fungal activity reflected by inhibition of the yeast. Thus, the selection criteria provided by *Streptomyces* 85E cannot be generally duplicated using other common organisms.

### EXAMPLE 4

Isolate 60A (believed to be a *Bacillus* species related by 16S rDNA sequence analysis to *Bacillus licheniformis*) which tested positive in Example 1 was grown in tryptic soy broth (Difco) for 24 to 48 hours. A crude cell-free supernatant was recovered by centrifugation at 17,000 X g for 10 minutes. This crude supernatant was sterilized by passage though a 0.2 micron filter and then used for further tests to evaluate inhibition of MAP kinase.

Tests for MAP kinase inhibition were performed using standard reagents and protocols equivalent to those supplied by from Upstate Biotechnology. 30 ul reactions mixtures were prepared containing MAPK enzyme, myelin basic protein as substrate and 5 ul of the crude supernatant. The reaction mixtures were incubated for 10 minutes at 30C with -³²P-ATP, and the radioactivity incorporated in the protein was determined by scintillation counting. In duplicate experiments, the activity in the sample containing the crude supernatant was found to be only 3.8% and 6.25% of the activity of a control. Thus, isolate 60A apparently produces an effective inhibitor of MAP kinase.

### EXAMPLE 5

The experiment of Example 4 was repeated on supernatants from eleven additional isolates that were positive in the sporulation inhibition assay. Of these eleven supernatants, a total of five were effective to inhibit MAP Kinase. As shown in Table 1, this activity persisted even when the crude preparation was diluted 10-fold.

**TABLE 1**

| Isolate | %Activity -Crude Supernatant | % Activity - 10-fold Dilution |
|---|---|---|
| Control | 100 | |
| 60A (putative *Bacillus)* | 2 | 14.5 |
| 152-O (putative *Bacillus)* | 2 | 37.5 |
| 11C (putative *Pseudomonas)* | 42 | 73 |

### EXAMPLE 6

To confirm that the inhibition in the MAP kinase assay was caused by a low molecular weight material and was not an artifact arising from the presence of a protease or ATPase, the crude supernatants were filtered through a filter unit with a molecular weight cut-off of 10,000 daltons. The filtered supernatant was then tested for its ability to inhibit MAP kinase. Supernatant from an isolate (31C) which showed no activity in the sporulation test of Example 1 was also run as an additional control. The results are shown in Table 2. As can be seen, the inhibitory activity remained in the filtrates, although the activity was somewhat reduced. The supernatant from isolate 31C did not inhibit the MAP kinase activity.

Efforts to isolate the active inhibitory compound from the 60A supernatant have not produced an identifiable compound which will inhibit MAP kinase activity. Thus, it remains possible that the activity detected is that of a protease which could pass through the 10,000 dalton filter, although most proteases are larger than 10,000 daltons in size. A more plausible explanation for the failure to identify a specific compound would be retention of the MAP kinase inhibitor in the separation media used to date or destruction/inactivation of the inhibitor by the isolation procedures attempted. In this case, the identified supernatants would still stand as the only known examples of MAP kinase-specific inhibitors.

**Table 2**

| Sample | % Activity, Crude | % Activity, Filtered |
|---|---|---|
| Control | 100 | |
| 60A | 62 | 83 |
| 152-O | 64 | 74 |
| 11C | 58 | 89 |
| 31C | 97 | n.d. |

### EXAMPLE 7

The crude and filtered supernatant from isolate 60A was tested for its ability to inhibit the tyrosine kinase, src, using commercially available reagents and protocols. The activity observed for the crude supernatant was 61% percent of the control while that for the filtrate was 67% of the control. Thus, the supernatant from 60A also inhibited tyrosine kinase.

### EXAMPLE 8

The crude supernatant from isolate 60A was tested for its ability to inhibit the serine/threonine kinase, cdc2, using reagents and protocols such as those supplied by Upstate Biotechnology. The activity observed for the crude supernatant was 99% percent of the control. Thus, the supernatant from 60A does not inhibit serine/threonine kinase.

### EXAMPLE 9

Marked inhibition of four additional wild strains (17A, 31C, 68A and 73F) was observed in tests using a number of the supernatants found to inhibit sporulation of strain 85E. 16S rDNA sequence data demonstrates a 40 base pair region in which considerable sequence variability has been noted. Comparison of the these sequences with those in GenBank suggest that these strains are probably four different species of *Streptomyces* and that all are different from Strain 85E and from *Streptomyces griseus.*

### EXAMPLE 10

As described in Example 1, cell-free supernatants prepared from cultures of soil isolates were applied to discs for testing for inhibition of sporulation of various tester strains. The supernatants from cultures of two isolates, designated WEC64-11C and WEC297-60A (or 11C and 60A) were among those which have tested positive as determined by observance of a zone around the disc in which growth of vegetative mycelia was evident but development of aerial mycelia and sporulation had been inhibited. The diameter of this inhibition zone varies with different preparations of the cell cultures but typically is in the range of 20 to 25 mm using the tester strain Streptomyces 85E. These supernatants also inhibit sporulation of other species of Streptomyces including *Streptomyces griseus* and wild strains 17A and 31C (Examples 2 and 9).

### EXAMPLE 11

For isolation of the active inhibitor from the supernatant of *Bacillus* 60A, a 3 litre culture of *Bacillus* 60A was grown in TSB (tryptic soy broth, Difco) for 24 hours at 30°C from a 1% v/v inoculum from a freshly grown seed culture. Cells were harvested by centrifugation at 7880 x g and both the supernatant and cell pellet were extracted separately with 3 volumes of ethyl acetate and 3 volumes of 20% methanol in ethyl acetate, respectively. The crude ethyl acetate extracts were fractionated on a low mesh silica gel column in an increasing methanol gradient in chloroform. Inhibitory activity in the fractions was monitored by thin layer chromatography of aliquots on silica gel. Following development in chloroform:methanol (95:5 v/v) plates were transferred to square culture dishes and overlaid with ISP (Difco) soft agar (0.6 %) containing an inoculum of 10⁷-10⁸ cfu from a fresh culture of *Streptomyces* 85E. After incubation at 30°C for 24 to 30 hours fractions with activity could be identified by inhibition of sporulation in a zone over one or more spots. Using this method an active fraction eluting at 12% methanol in chloroform was identified and further purified by column chromatography on Sephadex LH-20 in chloroform:methanol (1:1v/v). Once again the active fraction was identified and chromatographed once more on Sephadex LH-20, this time in chloroform:methanol 8:2 v/v. 44.2 mg of a pure, active compound designated TDI-4 were recovered. TDI-4 is a white powder, soluble in chloroform, which develops as a pink spot when sprayed with vanillin-H₂SO₄ following TLC in chloroform:methanol: acetone, 4:3:3 v/v. At this stage it was subjected to low resolution FAB-mass spectroscopy which gave M⁺ at m/z 1036. High resolution FAB-MS suggested the formula C₅₃H₉₃N₇O₁₃. Given the source of this compound, its molecular weight and probable formula an identification as the peptide surfactin seemed likely. Amino acid analysis yielded a composition of leucine x 4, aspartic acid x 1, glutamic acid x 1 and valine x 1, a composition consistent with surfactin. TDI-4 was then directly compared to surfactin from *Bacillus subtilis* (obtained from Sigma, molecular weight 1036) by co-TLC and by NMR spectroscopy and was found to match.

### EXAMPLE 12

For isolation of the active inhibitor from *Pseudomonas* 11C, a 10 litre culture of *Pseudomonas* 11C was grown in tryptic soy broth for 24 hours at 30°C from a 1% v/v inoculum from a freshly grown seed culture. Cells were harvested by centrifugation at 7880 x g and both the supernatant and cell pellet were extracted separately with ethyl acetate and 20% methanol in ethyl acetate, respectively. As before, the crude ethyl extracts were fractionated initially on a silica gel column in chloroform, followed by further chromatography of active fractions (identified by TLC and an overlay bioassay as described previously) on Sephadex LH-20 in methanol. 4.2 mg of a pure active compound designated TDI-5 were recovered. TDI-5 is a yellowish white compound which also develops as a pink spot on a TLC plate when developed in chloroform:methanol:acetone (6:2:2) and sprayed with vanillin-H₂SO₄. The LRFAB-MS analysis suggested a molecular weight of 1126. The molecular formula for m/z 1126 was determined to be C₅₄H₉₆N₉O₁₆ by HRFAB-MS analysis. Based on this information and the source of the compound, an identification as the peptide viscosin seemed likely and amino acid analysis yielded a composition of leucine x 3., valine x 1, serine x 2, isoleucine x 1 and glutamic acid x 1. The presence of a ninth amino acid as D-allo-threonine was demonstrated by the mass fragmentation data calculated from LRFAB-MS. TDI-5 was then compared to an authentic sample of viscosin (a gift from Raymond Anderson) by co-TLC and found to match.

### EXAMPLE 13

Testing the purified surfactin and viscosin was carried out to determine a) their ability to inhibit sporulation of Streptomyces species and b) their ability to inhibit the activity of eukaryotic protein kinases.

### a) Inhibition of sporulation:

Samples of surfactin prepared as described from *Bacillus* 60A as well as surfactin from *Bacillus subtilis* with the same molecular weight (1036) obtained from Sigma were tested for their ability to inhibit sporulation of *Streptomyces* 85E.

| **Amount of surfactin applied to the disc** | **Diameter of zone of inhibition of sporulation** |
|---|---|
| 1 ug, Sigma surfactin | 12 mm |
| 5 ug, Sigma surfactin | 15 mm |
| 10 ug, Sigma surfactin | 20 mm |
| | |
| 1 ug surfactin from Bacillus 60A | 13 mm |
| 5 ug surfactin from Bacillus 60A | 17 mm |
| 10 ug surfactin from Bacillus 60A | 19 mm |

Surfactin in these concentrations does not inhibit growth of the vegetative mycelia but inhibits development of aerial mycelia and spore formation. The effect is very persistant, lasting for at least 3 to 4 days after unaffected portions of the culture have sporulated. Eventually the inhibition is overcome and the culture sporulates . It has also been noted that surfactin in these concentrations does not inhibit growth of other bacterial species such as *E. coli* or *S. aureus.* However, surfactin has been reported to be have antimycobacterial activity (Stachelhaus *et al.*, 1996) and this has been confirmed with tests of these samples on *Mycobacterium phlei.* (25 ug Sigma surfactin applied to a disc produced a 12 mm zone of growth inhibition and 20 ug of surfactin from *Bacillus* 60A produced an 8 mm zone of growth inhibition.) The mechanism of this inhibition is not known; however, Mycobacteria have also been shown to possess certain elements of eukaryotic signal transduction pathways (Av-Gay and Davies, 1997) and it is possible that surfactin, acting as a kinase inhibitor, is interfering with signaling processes in this organism. It is further possible that the screen described which potentially detects inhibitors of eukayotic signaling processes may also have potential in the discovery of novel antimycobacterial agents. At least 8 cell-free supernatants which were found to inhibit sporulation of *Streptomyces* 85E also have antimycobacterial activity in tests of inhibition of *M. aurum ,M. phlei* and *M. tuberculosis* but do not inhibit growth of *S. aureus* or *E. coli.* These active supernatants are from two species of Streptomyces, a Bacillus species and a Pseudomonas species isolated from soils. The active compounds from the Streptomyces sp. have not yet been purified and identified. The Bacillus species are related to 60A and are probably producing surfactin while the Pseudomonad is a viscosin producer.

Viscosin purified from *Pseudomonas* 11C was also shown to inhibit sporulation of *Streptomyces* 85E (15 ug produces a 30 mm zone of persistent sporulation inhibition with no inhibition of growth of vegetative mycelia) Viscosin has also been reported to have antimycobacterial activity (Gerared et al., 1997) and this was confirmed with inhibition of growth of *M. aurum*. (25 ug applied to a disc resulted in a 9 mm zone of growth inhibition.)

### b) Inhibition of eukaryotic protein kinases:

### I) Inhibition of the protein tyrosine kinase c-Src:

Human c-Src (p60^{c-src}) was obtained from Upstate Biotechnology. The activity of the enzyme was assayed in an ELISA based assay developed by Pierce. Following their protocol, 500 ng of a biotinylated peptide substrate in distilled water (Pierce biotinylated peptide substrate #2, sequence Biotin-EGPWLEEEEEAYGWMDF) was added to a NeutrAvidin™ coated well, incubated at 37°C for 30 minutes to allow binding to the well then washed with Tris-buffered saline (TBS, 25mM Tris, 0.15 M NaCl, pH 7.6). For each reaction, a reaction buffer mixture was prepared to contain 10 ul of src assay buffer (100 mM Tris-HCl, pH 7.2, 25mM MnCl₂, 2.0 mM EGTA, 0.25 mM Na₃VO₄, 125mM Mg Acetate) 20 ul of ATP/MgCl₂ buffer (5 mM ATP, 50 mM MgCl₂ in TBS) and 1 ul of protease inhibitor mix (PMSF and pepstatin, final concentrations in Rx: 0.5 mM and 1.5uM). The src enzyme is supplied at a concentration of 3 units per microlitre and dilutions of the kinase were prepared in 1% bovine serum albumin (BSA) in TBS typically to final concentrations of 0.2 or 0.4 units in 10 ul. 10 ul of a solution of a compound to be tested for inhibitory activity was added to the reaction buffer mixture and allowed to stand 10-15 min. at room temperature, then 10 ul of the diluted kinase enzyme was added and the mixture was allowed to stand 10 min. at room temperature before addition to the substrate coated well. The plate was incubated at 30°C for varying periods of time then the contents were washed out with repeated rinsings with TBS. A mouse monoclonal anti-phosphotyrosine antibody (PY20) conjugated to horseradish peroxidase (Pierce) was diluted 1:2000 in 1% BSA diluent from 1 mg/ml and 75 ul of this dilution was added to each well, incubated 1 hour at 37°C then washed out with repeated rinsings with TBS.

100 ul of horseradish peroxidase substrate solution (3,3',5,5' tetramethylbenzidine and H₂O₂, Pierce TMB-ELISA) was added to each well and after 10 min. the reaction was stopped with the addition of 100 ul of 1 N H₂SO₄. Absorbance was read at 450 nm in a Molecular Devices V-max plate reader.

Examples of inhibition detected using this assay: (Values reported are the absorbance readings after subtraction of a blank well containing no enzyme.)

| I. Human c-Src kinase incubated 30 min. at 30°C with 10 ug of surfactin (srf) or 5 ug of viscosin (vis): | | |
|---|---|---|
| Control reactions | Reactions with surfactin | Reactions with viscosin |
| 0.05 unit c-Src | 0.05 unit c-Src + 10 ug srf | |
| 0.315 | 0.050 | |
| | | |
| 0.1 unit c-Src | 0.1 unit c-Src + 10 ug srf | 0.1 unit c-Src + 5 ug vis |
| 0.391 | 0.093 | 0.186 |
| | | |
| 0.2 unit c-Src | 0.2 unit c-Src + 10 ug srf | 0.2 unit c-Src + 5 ug vis |
| 0.396 | 0.191 | 0.175 |
| | | |
| 0.4 unit c-Src | 0.4 unit c-Src + 10 ug srf | 0.2 unit + 5 ug vis |
| 0.517 | 0.311 | 0.251 |

| II. Human c-Src kinase incubated 5 min. at 30°C with surfactin or viscosin: | | |
|---|---|---|
| Control | | |
| 0.2 unit c-Src | 0.2 unit c-Src + 10 ug srf | 0.2 unit c-src + 5 ug vis |
| 0.220 | 0.087 | 0.123 |
| | | |
| | 0.2 unit c-Src + 5 ug srf | 0.2 unit c-Src + 2.5 ug vis |
| | 0.185 | 0.210 |

10 ug of surfactin added to these reactions represents a concentration of 200 uM in the reaction and this concentration as well as 100 uM has inhibited the activity of the tyrosine kinase tested. Similarly viscosin at a concentration of 90 uM has inhibited the activity of this kinase. The preparations of the inhibitors from the soil bacterial isolates as well as the Sigma preparation of surfactin have been tested for residual protease activities by various methods including:
a. incubation with the chromogenic substrate N-succinyl-ala-ala-pro-phe p-nitroanilide
b. incubation with a mixture of proteins followed by SDS-PAGE analysis
c. zymogram analysis in a gel containing gelatin as the protease substrate
The crude culture supernatant from the Bacillus clearly contains protease activity but none was detectable in the purified preparations of surfactin. The Pseudomonas culture supernatant contains a lesser amount of protease activity and again none was detected in the purified preparation of viscosin. Both of these compounds are surfactants and the possibility that the apparent inhibition was due to interference with either the avidin-biotin interaction or the adherence of the avidin to the wells was tested by preincubating the substrate coated well with a reaction mixture containing surfactin or viscosin but no enzyme for 30 minutes. The well was then washed with repeated rinsings of TBS and a standard kinase reaction without the inhibitors was set up. This reaction showed no decrease in activity compared to a control reaction. Thus, it is concluded that surfactin and viscosin are inhibitors of this tyrosine kinase.

### ii) Inhibition of the phospho-lipid dependent serine-threonine kinase Protein Kinase C (PKC)

Protein Kinase C purified from rat brain was obtained from Upstate Biotechnology. The activity of the enzyme was assayed in a colorimetric assay developed by Pierce. Reaction mixtures were prepared to contain 5 ul each of the 5X reaction buffer, the dye labeled glycogen synthase synthetic peptide substrate reconstituted to 1.77 mM and the 5X activator solution of phosphatidyl-L-serine, all supplied by Pierce in a PKC assay kit. The activator solution was sonicated for 30 sec. on ice prior to use. 1 ul of the kinase (equivalent to 10 ng of enzyme with a specific activity of 12.1 units/mg) was added to 10 ul of a solution containing the compound to be tested for inhibitory activity and allowed to stand at room temperature for 10 minutes then transferred to the reaction tube containing the substrate and incubated 30 min. at 30°C. After incubation 20 ul of each sample was applied to the centre of the affinity membrane in the separation units supplied with the assay kit. These membranes specifically bind the phosphorylated dye labeled substrate while non-phosphorylated peptide will pass through when spun at low speeds. After washing the membranes the phosphorylated peptide was eluted by spinning with the elution buffer and absorbance read at 570 nm in a Molecular Devices V-max plate reader.

Viscosin at a concentration of 200 uM in the reaction was not inhibitory. Surfactin at a concentration of 380 uM in the reaction was only minimally inhibitory as shown in the following example:

| Control reaction | |
|---|---|
| 10 ng PKC | 10 ng PKC + 10 ug srf |
| 0.228 | 0.181 |

Polymyxin B is a peptide with a molecular weight similar to viscosin and surfactin and is a recognized inhibitor of PKC. Inhibition of the kinase could be detected in this assay system as shown by the following example:

| Control reaction | |
|---|---|
| 10 ng PKC | 10 ng PKC + 10 ug polymyxin B |
| 0.203 | 0.035 |

Thus it was concluded that surfactin and viscosin are more potent inhibitors of the tyrosine kinase tested than this serine-threonine kinase.

### EXAMPLE 14

Extracts (in methanol:acetone, 1:1) were prepared from 62 species of lichens and 52 extracts were found to inhibit sporulation of the tester strain *Streptomyces* 85E. Prior to initiation of attempts to purify active compounds from many different lichen species, two widely distributed lichen compounds were tested: namely vulpinic acid and usnic acid (both obtained from Sigma).

| **Inhibition of sporulation in Streptomyces 85E** | |
|---|---|
| Compound applied to disc | Inhibition zone |
| 10 ug usnic acid | 15 mm |
| 10 ug vulpinic acid | 15mm |

Growth of the vegetative mycelia was not inhibited. The inhibition of sporulation was persistent, lasting for at least 2 to 3 days. The presence of usnic acid and vulpininc acid in several of the positive lichen extracts was confirmed by co-TLC with the authenticated compounds followed by an agar overlay test with *Streptomyces* 85E. Sporulation was inhibited in zones over the spots of these compounds and also over spots with the same _{R}F value in the lichen extracts. At least 16 of the extracts were found to contain usnic acid and at least 5 contain vulpinic acid.

Examples of inhibition of protein kinase activities:

| I. Human c-Src kinase incubated 30 min. at 30°C with 5 ug usnic acid or vulpinic acid: | | |
|---|---|---|
| Control reaction | | |
| 0.2 unit c-Src | 0.2 unit c-Src + usnic acid | 0.2 unit c-Src + vulpinic |
| acid | | |
| 0.562 | 0.417 | 0.483 |

| II. Human c-Src incubated 5 min. at 30°C with 5 ug usnic acid or 5 ug vulpinic acid: | | |
|---|---|---|
| Control reaction | | |
| 0.2 unit c-Src | 0.2 unit c-Src + usnic acid | 0.2 unit c-Src + vulpinic |
| acid | | |
| 0.208 | 0.120 | 0.180 |

| III. Protein Kinase C incubated 30 min. at 30°C with 5 ug usnic acid or 5 ug vulpinic acid: | | |
|---|---|---|
| Control reaction | | |
| 10 ng PKC | 10 ng PKC + usnic acid | 10 ng PKC + vulpinic acid |
| 0.289 | 0.234 | 0.230 |

5 ug in the 50 ul PTK reaction mixture represents a concentration of about 300 uM for both of these compounds and twice that in the 25 ul PKC reactions. Thus, some inhibition was detected; however, these compounds appear to be less inhibitory than surfactin or viscosin for this particular protein tyrosine kinase and only minimally inhibitory for protein kinase C.

## Claims

1. A method for assaying a material for a previously unrecognized activity as an inhibitor of eukaryotic post-translational protein modification, calcium signal modulation, cell-cycle development or apoptosis comprising
adding the material to a growing culture of a prokaryotic organism which possesses enzyme activity effective to phosphorylate tyrosine, serine or threonine residues within a protein;
allowing the culture to grow for a period of time in the presence of the material; and
observing the culture for altered development relative to development of the prokaryotic organism grown in the absence of the material, wherein altered development is indicative that the material has activity as an inhibitor of post-translational protein phosphorylation.

2. The method according to claim 1, wherein the material is added to a growing culture of the prokaryotic organism by placing a carrier disk bearing the material on a freshly seeded plate.

3. The method according to claim 1, wherein the prokaryotic organism is a streptomycete.

4. The method according to claim 3, wherein the culture is observed for the inhibited development of aerial mycelia and spore formation as an indicator that the material has activity as an inhibitor of post-translational protein phosphorylation

5. The method according to claim 3, wherein the streptomycete is a strain of *Streptomyces griseus*.

6. The method according to claim 3, wherein the streptomycete is *Streptomyces* 85E (ATCC No. 55824).

7. A method for assaying a material for a previously unrecognized activity as an inhibitor of eukaryotic enzymes comprising
adding the material to a growing culture of a prokaryotic organism which possesses enzyme activity effective to phosphorylate tyrosine, serine or threonine residues within a protein;
allowing the culture to grow for a period of time in the presence of the material; and
observing the culture for altered development relative to development of the prokaryotic organism grown in the absence of the material, wherein altered development is indicative that the material has activity as an enzyme inhibitor.

8. The method according to claim 7, wherein the material is added to a growing culture of the prokaryotic organism by placing a carrier disk bearing the material on a freshly seeded plate.

9. The method according to claim 7, wherein the prokaryotic organism is a streptomycete.

10. The method according to claim 9, wherein the culture is observed for the inhibited development of aerial mycelia and spore formation as an indicator that the material has activity as an inhibitor of post-translational protein phosphorylation

11. The method according to claim 9, wherein the streptomycete is a strain of *Streptomyces griseus*.

12. The method according to claim 9, wherein the streptomycete is *Streptomyces* 85E (ATCC No. 55824).

13. The method according to any of claims 1 to 12, wherein the material being tested is a cell-free preparation taken from a culture of microorganism or a lichen extract.

14. The method according to claim 13, further comprising the step of isolating an active inhibitory compound from the culture of microorganism or lichen.

15. A substantially pure culture of *Streptomyces* 85E (ATCC No. 55824).

## Patentansprüche

1. Verfahren zum Untersuchen eines Materials auf eine zuvor nicht bemerkte Aktivität als Inhibitor für eukaryotische post-translationale Proteinmodifikation, Calciumsignalmodulation, Zellzyklusentwicklung oder Apoptosis, umfassend:
Zugeben des Materials zu einer wachsenden Kultur aus prokaryotischem Organismus, welcher eine wirksame Enzymaktivität besitzt, um Tyrosin-, Serin- oder Threonin-Reste innerhalb eines Proteins zu phosphorylieren;
Wachsenlassen der Kultur für eine Zeitperiode in Gegenwart des Materials; und
Beobachten der Kultur für eine veränderte Entwicklung relativ zur Entwicklung des in Abwesenheit des Materials gewachsenen prokaryotischen Organismus, worin die veränderte Entwicklung indikativ ist, dass das Material eine Aktivität als Inhibitor der post-translationalen Proteinphosphorylierung besitzt.

2. Verfahren nach Anspruch 1, worin das Material zu einer wachsenden Kultur von prokaryotischem Organismus zugegeben wird durch Anordnen einer Trägerscheibe, welche das Material auf einer frisch besamten Platte trägt.

3. Verfahren nach Anspruch 1, worin der prokaryotische Organismus ein Streptomycet ist.

4. Verfahren nach Anspruch 3, worin die Kultur beobachtet wird in Bezug auf die gehemmte Entwicklung von Luftmyzelien und Sporenbildung als Indikator, dass das Material eine Aktivität als ein Inhibitor für post-translationale Proteinphosphorylierung besitzt.

5. Verfahren nach Anspruch 3, worin das Streptomycet ein Stamm von *Streptomyces griseus* ist.

6. Verfahren nach Anspruch 3, worin das Streptomycet *Streptomyces* 85E (ATCC Nr. 55824) ist.

7. Verfahren zum Untersuchen eines Materials auf eine zuvor nicht bemerkte Aktivität als Inhibitor für eukaryotische post-translationale Proteinmodifikation, Calciumsignalmodulation, Zellzyklusentwicklung oder Apoptosis, umfassend:
Zugeben des Materials zu einer wachsenden Kultur aus prokaryotischem Organismus, welcher eine wirksame Enzymaktivität besitzt, um Tyrosin-, Serin- oder Threonin-Reste innerhalb eines Proteins zu phosphorylieren;
Wachsenlassen der Kultur für eine Zeitperiode in Gegenwart des Materials; und
Beobachten der Kultur für eine veränderte Entwicklung relativ zur Entwicklung des in Abwesenheit des Materials gewachsenen prokaryotischen Organismus, worin die veränderte Entwicklung indikativ ist, dass das Material eine Aktivität als Inhibitor der post-translationalen Proteinphosphorylierung besitzt.

8. Verfahren nach Anspruch 7, worin das Material zu einer wachsenden Kultur von prokaryotischem Organismus zugegeben wird durch Anordnen einer Trägerscheibe, welche das Material auf einer frisch besamten Platte trägt.

9. Verfahren nach Anspruch 7, worin der prokaryotische Organismus ein Streptomycet ist.

10. Verfahren nach Anspruch 9, worin die Kultur beobachtet wird in Bezug auf die gehemmte Entwicklung von Luftmyzelien und Sporenbildung als Indikator, dass das Material eine Aktivität als ein Inhibitor für post-translationale Proteinphosphorylierung besitzt.

11. Verfahren nach Anspruch 9, worin das Streptomycet ein Stamm von *Streptomyces griseus* ist.

12. Verfahren nach Anspruch 9, worin das Streptomycet *Streptomyces* 85E (ATCC Nr. 55824) ist.

13. Verfahren nach einem der Ansprüche 1 bis 12, worin das zu testende Material eine zellfreie Präparation, genommen aus einer Kultur von Mikroorganismen oder einem Flechtenextrakt, ist.

14. Verfahren nach Anspruch 13, weiterhin umfassend den Schritt der Isolierung einer aktiven Hemmverbindung aus der Kultur von Mikroorganismus oder Flechte.

15. Weitgehend reine Kultur von *Streptomyces* 85E (ATCC Nr. 55824).

## Revendications

1. Procédé de détermination dans une substance d'une activité non identifiée antérieurement d'inhibiteur de modification post-traductionnelle de protéine d'eucaryote, de modulation du message calcique, de développement du cycle cellulaire ou d'apoptose, comprenant des étapes consistant à
ajouter la substance à une culture en croissance d'un organisme procaryotique qui possède une activité enzymatique apte à phosphoryler des résidus tyrosine, sérine ou thréonine dans une protéine ;
laisser la culture croître pendant une période de temps en présence de la substance ; et
rechercher dans la culture un développement modifié relatif au développement de l'organisme procaryotique cultivé en l'absence de la substance, **caractérisé en ce qu'**un développement modifié est une indication que la substance a une activité en tant qu'inhibiteur de phosphorylation post-traductionnelle de protéine.

2. Procédé selon la revendication 1, **caractérisé en ce que** la substance est ajoutée à une culture en croissance de l'organisme procaryotique en disposant un disque support portant la substance sur une plaque fraîchement ensemencée.

3. Procédé selon la revendication 1, **caractérisé en ce que** l'organisme procaryotique est un streptomycète.

4. Procédé selon la revendication 3, **caractérisé en ce qu'**on recherche dans la culture le développement inhibé de mycélia aériens et de formation de spores comme indication que la substance a une activité en tant qu'inhibiteur de phosphorylation post-traductionnelle de protéine.

5. Procédé selon la revendication 3, **caractérisé en ce que** le streptomycète est une souche de *Streptomyces griseus*.

6. Procédé selon la revendication 3, **caractérisé en ce que** le streptomycète est *Streptomyces* 85E (ATCC N°55824).

7. Procédé de détermination dans une substance d'une activité non identifiée antérieurement d'inhibiteur d'enzymes eucaryotiques comprenant des étapes consistant à
ajouter la substance à une culture en croissance d'un organisme procaryotique qui possède une activité enzymatique apte à phosphoryler des résidus tyrosine, sérine ou thréonine dans une protéine ;
laisser la culture croître pendant une période de temps en présence de la substance ; et
rechercher dans la culture un développement modifié relatif au développement de l'organisme procaryotique cultivé en l'absence de la substance, **caractérisé en ce qu'**un développement modifié est une indication que la substance a une activité en tant qu'inhibiteur d'enzyme.

8. Procédé selon la revendication 7, **caractérisé en ce que** la substance est ajoutée à une culture en croissance de l'organisme procaryotique en disposant un disque support portant la substance sur une plaque fraîchement ensemencée.

9. Procédé selon la revendication 7, **caractérisé en ce que** l'organisme procaryotique est un streptomycète.

10. Procédé selon la revendication 9, **caractérisé en ce qu'**on recherche dans la culture le développement inhibé de mycélia aériens et de formation de spores comme indication que la substance a une activité en tant qu'inhibiteur de phosphorylation post-traductionnelle de protéine.

11. Procédé selon la revendication 9, **caractérisé en ce que** le streptomycète est une souche de *Streptomyces griseus*.

12. Procédé selon la revendication 9, **caractérisé en ce que** le streptomycète est *Streptomyces* 85E (ATCC N°55824).

13. Procédé selon l'une quelconque des revendications 1 à 12, **caractérisé en ce que** la substance testée est une préparation acellulaire issue d'une culture d'un microorganisme ou d'un extrait de lichen.

14. Procédé selon la revendication 13, comprenant en outre l'étape consistant à isoler un composé inhibiteur actif de la culture de microorganisme ou de lichen.

15. Culture substantiellement pure de *Streptomyces* 85E (ATCC N°55824)
